(19) **Europäisches Patentamt**
**European Patent Office**
**Office européen des brevets**

(11) **EP 2 067 439 B1**

(12) **EUROPÄISCHE PATENTSCHRIFT**

(45) Veröffentlichungstag und Bekanntmachung des Hinweises auf die Patenterteilung:
**18.01.2012 Patentblatt 2012/03**

(51) Int Cl.:
***A61B 5/0488*** *(2006.01)*

(21) Anmeldenummer: **07400028.2**

(22) Anmeldetag: **06.12.2007**

(54) **Verfahren zum Bestimmen einer muskulären Ermüdung**

Method for determining muscular fatigue

Procédé de détermination de la fatigue musculaire

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**10.06.2009 Patentblatt 2009/24**

(73) Patentinhaber: **Thumedi GmbH & Co. KG**
**09419 Thum-Jahnsbach (DE)**

(72) Erfinder:
• **Seibt, Robert**
**09419 Thum-Jahnsbach (DE)**
• **Schneider, Jan**
**09423 Gelenau (DE)**

(74) Vertreter: **Steiniger, Carmen**
**Riarda-Huch-Str. 4**
**09116 Chemnitz (DE)**

(56) Entgegenhaltungen:
**EP-A- 1 859 737     US-A- 5 086 779**

• **HÄGG G M ET AL: "Methodologies for evaluating electromyographic field data in ergonomics." JOURNAL OF ELECTROMYOGRAPHY AND KINESIOLOGY : OFFICIAL JOURNAL OF THE INTERNATIONAL SOCIETY OF ELECTROPHYSIOLOGICAL KINESIOLOGY OCT 2000, Bd. 10, Nr. 5, Oktober 2000 (2000-10), XP002484022 ISSN: 1050-6411**

## Beschreibung

Die vorliegende Erfindung betrifft ein Verfahren zum Bestimmen einer muskulären Ermüdung aus elektromyographischen Messwerten.

Die Elektromyographie ist eine etablierte Methode zur Beurteilung von Muskelaktivitäten. Allgemein versteht man unter der Elektromyographie ein Verfahren zur Ermittlung des Erregungs- und Kontraktionszustandes der Skelettmuskulatur. Bei der Elektromyographie (EMG) bzw. der Oberflächenmyographie (OEMG) werden in der Regel Oberflächenelektroden verwendet, die über dem Muskel auf der Haut angebracht werden. Die Signale des EMG werden üblicherweise mit bipolaren Ableitungen erfasst, das heißt, es wird die Spannungsdifferenz zwischen zwei Elektroden in Bezug auf eine Referenzelektrode ermittelt. Erfasst werden dabei die bei der Fortleitung von Aktionspotentialen an der Muskelfasermembran erzeugten Potentialänderungen. Das gemessene EMG-Signal stellt damit ein extrazellulär abgeleitetes Summensignal aller Aktionspotentiale der aktiven motorischen Einheiten dar.

Ausgehend von den EMG-Rohsignalen, welche bereits eine erste qualitative Betrachtung des Aktivierungsverhaltens des untersuchten Muskels erlauben, können zeit- und amplitudenbezogene Auswertungen vorgenommen werden. Zur Bestimmung der Aktivitätshöhe werden meist Mittelwerte oder die so genannten root mean square (rms)-Werte (Wurzel aus dem Mittelwert der quadrierten Amplitudenwerte) über einen zu definierenden Kurvenabschnitt berechnet. Eine Methode zur Bestimmung von relativen Muskelaktivitäten ist die Normierung der bei einer Bewegung erzeugten elektrischen Aktivität eines Muskels aus der Höhe seiner Aktivität bei einer willkürlichen maximalen Muskelkontraktion (MVC: maximal voluntary contraction). In einer zeitbezogenen Auswertung können neben Aktivitätsbeginn und -ende auch die Zeitpunkte der maximalen Muskelaktivität und die Dauer von Aktivitätsbeginn bis zur maximalen Amplitude bestimmt werden. Aus einem geglätteten und gleichgerichteten EMG-Signal lassen sich neben der elektrischen Aktivität und dem Effektivwert (root mean square, rms) weitere Kennwerte wie die Bandleistung innerhalb eines definierten Frequenzbandes ermitteln.

Ferner können die ermittelten elektromyographischen Rohsignale in ein Frequenzspektrum transformiert werden. Aus dem Frequenzspektrum eines EMG-Signals können in der Fachwelt allgemein anerkannte Kennwerte, wie die Medianfrequenz (median frequency), die Mittelfrequenz (mean frequency), einzelne oder mehrere Frequenzbänder usw. erhalten werden, welche zur Untersuchung der muskulären Ermüdung verwendet werden können.

Der muskuläre Kraftaufwand kann als absolute Kraft oder, was üblicher ist, als relative Kraft dargestellt werden. Die von einem Muskel zu einem bestimmten Zeitpunkt aufgebrachte relative Kraft ist dabei das Verhältnis zwischen angewandter Kraft und der maximalen willkürlichen Kontraktionskraft (maximal voluntary contraction, MVC) des Muskels. An einem während der Messung nicht ermüdeten bzw. zwischen den Messungen hinreichend erholten Muskel besteht zwischen relativer Muskelkraft und mehreren EMG-Kennwerten ein konstanter und eindeutiger, teilweise sogar eineindeutiger Zusammenhang. Beispielsweise besteht nach einer geeigneten Kalibrierung, in welcher die maximale willkürliche Kontraktionskraft bestimmt und der Kraftaufwand und ein gewählter Kennwert am nicht ermüdeten und nicht ermüdenden Muskel synchron gemessen wird, ein eineindeutiger Zusammenhang zwischen relativer muskulärer Kraft und elektrischer Aktivität, welcher eine eindeutige Ermittlung der Kraft aus dem EMG ermöglicht. Voraussetzung ist jedoch, dass die Muskulatur gegenüber der Kalibrierung während der Messung und zwischen den Messungen nicht ermüden darf.

Grundsätzlich gilt, dass alle aus einer elektromyographischen (EMG) bzw. speziell einer oberflächenelektromyographischen (OEMG) Messung abgeleiteten Kennwerte sowohl von der aufgewendeten Muskelkraft als auch von der aktuellen Ermüdung des bemessenen Muskels abhängig sind. Beispielsweise nimmt die elektrische Aktivität mit zunehmender muskulärer Ermüdung und auch mit zunehmendem Kraftaufwand zu. Die Medianfrequenz nimmt mit zunehmender muskulärer Ermüdung ab und mit zunehmendem Kraftaufwand zu.

Der Artikel von Hägg, Luttmann und Jäger: "Methodologies for evaluating electromyographic field data in ergomomics", Journal of Electromyography and Kinesiology 10 (2000) 301-312 beschreibt unter anderem das JASA-Verfahren (Joint Analysis of EMG Spectrum and Amplitude). In dem JASA-Verfahren werden gleichzeitig Änderungen der EMG-Amplitude und des Frequenzspektrums über der Zeit betrachtet, eine lineare Regressionsanalyse der gemessenen Amplituden- und Frequenzwerte durchgeführt, Wertepaare der jeweiligen Anstiege der Regressionskurven der Amplituden- und Frequenzwerte aufgenommen und diese in ein x-y-Diagramm mit vier Quadranten eingetragen. Anhand der Positionen der Messwerte innerhalb eines der vier Quadranten des Diagramms können die Messwerte in die Kategorien "Ermüdung", "Erholung", "Kraftanstieg" oder "KraftVerringerung" eingeordnet werden.

Nachteilig an den im Stand der Technik bekannten Verfahren zur Ermittlung der muskulären Ermüdung ist, dass dann, wenn sich Kraftaufwand und muskuläre Ermüdung gleichzeitig ändern, was der typische Fall im realen Leben ist, bisher keine Methode existiert, die es ermöglicht, die aufgewendete Kraft und die muskuläre Ermüdung getrennt voneinander zu bestimmen.

Auch erlauben die oben genannten Kennwerte nur eine semiquantitative Bestimmung der muskulären Ermüdung, so dass beispielsweise eine geringe oder eine starke Ermüdung erkannt werden kann. Bisher ist keine Methode bekannt, mit welcher eine Ermüdung exakt quantifizierbar ist, so dass beispielsweise die muskuläre Ermüdung auf einer Skala von 0 bis 100% einstufbar wäre. Da der muskuläre Ermüdungszustand und damit dessen Einfluss auf die oben genannten Kennwerte bisher aus dem EMG nicht quantitativ ermittelt werden kann, ist infolge auch die Bestimmung der aufgewendeten Kraft aus dem EMG bei variierender Ermüdung nicht möglich.

Ferner können alle aus dem EMG ermittelbaren Kennwerte, wie die Amplitude, die elektrische Aktivität, die Bandleistung, die Medianfrequenz, die Mittelfrequenz, die Phasenlage usw., auf Grund inter- und intraindividueller Unterschiede bei den bekannten Verfahren niemals direkt zwischen verschiedenen Probanden und auch nicht zwischen verschiedenen Muskeln eines Probanden verglichen werden. So kann beispielsweise die gemessene elektrische Aktivität bei gleicher Beanspruchung zweier Muskeln verschieden groß sein.

Es ist daher die Aufgabe der vorliegenden Erfindung, ein Verfahren zur Verfügung zu stellen, welches eine quantitative Bestimmung der muskulären Ermüdung, unabhängig von der aufgewendeten Kraft, vom analysierten Muskel und vom untersuchten Individuum, ermöglicht.

Diese Aufgabe wird durch ein Verfahren zum Bestimmen einer muskulären Ermüdung aus elektromyographischen Messwerten gelöst, wobei elektromyographische Rohsignale erfasst werden, aus den elektromyographischen Rohsignalen und/oder davon abgeleiteten Kennwerten wenigstens zwei elektromyographische Kennwerte ermittelt werden, wobei sich wenigstens zwei der elektromyographischen Kennwerte gleichsinnig bei variierender Kraft und gegensinnig bei variierender Ermüdung oder gegensinnig bei variierender Kraft und gleichsinnig bei variierender Ermüdung verhalten, in wenigstens einer ersten Kalibrierung ein funktionaler Zusammenhang zwischen den wenigstens zwei elektromyographischen Kennwerten ermittelt wird, wobei bei der Kalibrierung im Wesentlichen keine Muskelermüdung auftritt, und in einer Mess- und Auswertephase eine Differenz zwischen wenigstens einem aktuell ermittelten elektromyographischen Kennwert und dem funktionalen Zusammenhang zwischen den wenigstens zwei elektromyographischen Kennwerten bestimmt wird, ein funktionaler Zusammenhang zwischen einer bekannten oder durch Kalibrierung ermittelten muskulären Ermüdung und dieser Differenz ermittelt wird und daraus ein Wert für die tatsächliche muskuläre Ermüdung ermittelt wird.

Das erfindungsgemäße Verfahren ermöglicht es, eine muskuläre Ermüdung, insbesondere eine metabolische muskuläre Ermüdung, allein aus der Verknüpfung verschiedener Kennwerte, die aus elektromyographischen Messungen ermittelt werden können, quantitativ zu bestimmen. Das damit gemessene muskuläre Ermüdungsmaß ist unabhängig von der aufgewendeten Kraft, vom analysierten Muskel und vom untersuchten Individuum. Elektromyographische Rohsignale, vorzugsweise oberflächenelektromyographische Signale, können in der üblichen Weise am Muskel mittels Elektroden abgegriffen werden und direkt mit dem erfindungsgemäßen Verfahren verarbeitet werden.

Im Ergebnis ist es möglich, anhand der ermittelten elektromyographischen Signale die muskuläre Ermüdung des untersuchten Muskels in Form des Betrages der Verringerung der maximal möglichen willkürlichen Kontraktionskraft eines Muskels, beispielsweise auf 1 bis 100% normiert, oder in Form der nach einer muskulären Ermüdung bis zur vollständigen Erholung erforderlichen Erholzeit, beispielsweise auf 1 bis 100% normiert, darzustellen. Somit stehen im Ergebnis der vorliegenden Erfindung objektive, quantitative und vor allem universell vergleichbare Maße für die muskuläre Ermüdung zur Verfügung.

Entsprechend einer empfehlenswerten Variante der Erfindung werden die elektromyographischen Rohsignale in ein Frequenzspektrum transformiert werden und aus dem Frequenzspektrum wird wenigstens ein elektromyographischer Kennwert ermittelt und für die Ermittlung der muskulären Ermüdung herangezogen.

In einer vorteilhaften Ausgestaltung der vorliegenden Erfindung wird bei der wenigstens einen ersten Kalibrierung eine Kraftvariation am Muskel durchgeführt und es werden während der Kalibrierung wenigstens die zwei elektromyographischen Kennwerte und deren funktionaler Zusammenhang ermittelt, wobei bei der Kalibrierung im Wesentlichen keine Muskelermüdung auftritt. Auf diese Weise können die gemessenen elektromyographischen Kennwerte in Relation zu der Kraft am Muskel während der Kalibrierung dargestellt werden, bei welcher im Wesentlichen keine (zusätzliche) Muskelermüdung auftritt bzw. wobei zu Beginn der Kalibrierung die muskuläre Ermüdung entweder Null oder bekannt ist. Im folgenden sind diejenigen Werte, die in einem Zustand mit Muskelermüdung ermittelt werden, mit den in einem Zustand ohne Muskelermüdung ermittelten Werten zu vergleichen, Differenzen zwischen den Werten zu bilden und diese erfindungsgemäß zu nutzen, um die aktuelle Ermüdung des Muskels zu berechnen.

Gemäß einer bevorzugten Ausführungsform der vorliegenden Erfindung werden bei der Kraftvariation am Muskel alle zu analysierenden Muskeln gleichzeitig oder nacheinander mit einer stetig zunehmenden Kraft beansprucht und die wenigstens zwei elektromyographischen Kennwerte kontinuierlich oder wenigstens bei mehreren unterschiedlichen Kraftwerten ermittelt. Die Kraft selbst muss dabei nicht zwingend bekannt sein. Somit können die gemessenen beziehungsweise berechneten Kennwerte als Funktion der vergangenen Kalibrierzeit sowie als Funktion der Kraft dargestellt werden.

In einer besonders günstigen Variante der vorliegenden Erfindung wird bei der ersten Kalibrierung eine maximal vom Muskel aufzubringende Kraft so eingestellt, dass sie wenigstens der höchsten in einer anschließenden Messung auftretenden Kraft entspricht. Dadurch ist ein direkter Vergleich zwischen den in der Kalibrierung durchgeführten Messungen und Bedingungen am Muskel auch bei hohen auftretenden Kräften möglich.

Vorzugsweise wird bei der ersten Kalibrierung die vom Muskel relativ zu seiner maximalen willkürlichen Kontraktionskraft aufgebrachte Kraft kontinuierlich oder wenigstens bei mehreren unterschiedlichen Kraftwerten ermittelt. Auf diese Weise kann bei Verwendung des erfindungsgemäßen Verfahrens die muskuläre Ermüdung in Abhängigkeit von der relativen Muskelkraft, dass heißt im Verhältnis zu der maximalen willkürlichen Kontraktionskraft des Muskels, ermittelt werden.

In einem bevorzugten Beispiel der vorliegenden Erfindung wird dann, wenn die wenigstens zwei elektromyographischen Kennwerte nicht eineindeutig mit der Muskelkraft verknüpft sind, ein weiterer elektromyographischer Kennwert ermittelt und für eine eineindeutige Zuordnung zwischen Kennwert und Kraft verwendet. Mit der vorgeschlagenen Vorgehensweise kann an jedem Punkt der Kurve der ermittelten Kennwerte eine eindeutige Zuordnung von Kennwert zu Kraft und von Kraft zu Kennwert ermöglicht werden.

In einer favorisierten Variante der vorliegenden Erfindung wird aus den elektromyographischen Rohsignalen wenigstens eine Elektrische Aktivität und aus dem Frequenzspektrum wenigstens eine Medianfrequenz oder ein Amplitudenverhältnis zweier oder mehrerer Frequenzbänder ermittelt. Die aus den EMG-Rohsignalen ableitbare Elektrische Aktivität (EA) und die aus dem Frequenzspektrum ermittelbare Medianfrequenz (MF) verhalten sich bei Kraftvariationen gleichsinnig, das heißt, beide Kennwerte werden bei größer werdender Kraft ebenfalls größer, und bei Ermüdungsvariation gegensinnig, das heißt die Elektrische Aktivität nimmt mit zunehmender Ermüdung ebenfalls zu, während die Medianfrequenz mit zunehmender Ermüdung abnimmt. Entsprechend sind die Elektrische Aktivität und die Medianfrequenz besonders gut geeignet, um gemäß der vorliegenden Erfindung verarbeitet zu werden, um daraufhin eine muskuläre Ermüdung geeignet quantitativ erfassen zu können.

In einer besonders empfehlenswerten Ausführungsform der vorliegenden Erfindung wird eine zweite Kalibrierung durchgeführt, wobei in der zweiten Kalibrierung ein funktionaler Zusammenhang zwischen den wenigstens zwei elektromyographischen Kennwerten ermittelt wird, wobei der zu untersuchende Muskel definiert ermüdet wird, fortlaufend oder in diskreten zeitlichen Abständen eine Differenz ($\Delta$EMG) zwischen einem aktuell ermittelten elektromyographischen Kennwert und dem funktionalen Zusammenhang bestimmt wird, aus der Höhe der relativen Muskelkraft bei der Kalibrierbeanspruchung und der Dauer der Kalibrierbeanspruchung eine tatsächliche Muskelermüdung ermittelt wird, und ein funktionaler Zusammenhang zwischen der tatsächlichen Muskelermüdung und der Differenz ($\Delta$EMG) ermittelt wird. Während bei der ersten Kalibrierung mit der ermittelten Differenz $\Delta$EMG ein semiquantitatives Maß Ermüdung erfasst werden kann, gestattet eine Auswertung unter Zuhilfenahme der zweiten Kalibrierungswerte genaue quantitative Aussagen zur muskulären Ermüdung. Werden nur geringe Anforderungen an die Messgenauigkeit gestellt, kann grundsätzlich die zweite Kalibrierung entfallen. Stattdessen können durchschnittliche Transformationsparameter verwendet werden, sofern diese für den analysierten Muskel, die Elektrodenposition und die Art und Größe der Elektroden bekannt sind.

Die zusätzliche, zweite Kalibrierung erfüllt noch eine weitere Aufgabe: Die berechnete Differenz $\Delta$EMG ist bei angenommen konstanter muskulärer Ermüdung nicht unter allen Umständen vollständig unabhängig von der Variation der Muskelkraft. Ursache dafür ist die Nichtlinearität, vor allem die, im Vergleich der wenigstens zwei verwendeten EMG-Kennwerte unterschiedliche Nichtlinearität der Änderung der EMG-Kennwerte bei Ermüdung durch differente Kräfte. Die zusätzliche, zweite Kalibrierung kann die Kraftunabhängigkeit der Ermüdungsmessung in Abhängigkeit vom zu messenden Muskel und einer Reihe weiterer Faktoren verbessern.

Vorzugsweise werden in der zweiten Kalibrierung alle zu analysierenden Muskeln gleichzeitig oder nacheinander mit einer konstanten Kraft für eine definierte Zeit ermüdet, und während der Kalibrierung wenigstens die zwei elektromyographischen Kennwerte und deren funktionaler Zusammenhang in einem Zustand definierter Muskelermüdung ermittelt. Somit lassen sich unter der Voraussetzung, dass die zu analysierenden Muskeln vor Beginn der zweiten Kalibrierung vollständig erholt sind beziehungsweise ihre vorhandene Vorermüdung in ihrer Quantität bekannt ist, die elektromyographischen Kennwerte definiert bei Muskelermüdung erfassen. Entsprechend können die später, bei einer realen Messung am Muskel ermittelten elektromyographischen Kennwerte in Relation zu den bei definierter muskulärer Ermüdung erfassten elektromyographischen Kennwerten gebracht werden, woraufhin die muskuläre Ermüdung besonders zuverlässig und genau ermittelt werden kann.

Dabei ist es besonders von Vorteil, wenn die wenigstens zwei elektromyographischen Kennwerte kontinuierlich oder in definierten zeitlichen Abständen ermittelt werden. Somit können die bei definierter Muskelermüdung ermittelten elektromyographischen Kennwerte zeitabhängig erfasst werden. Entsprechend ist es möglich, dass aufgrund der Zeitabhängigkeit der erfassten EMG-Kennwerte diese auch zueinander in ein zeitunabhängiges Verhältnis gebracht werden können.

Hierbei sollte berücksichtigt werden, dass bei der zweiten Kalibrierung die vom Muskel aufzuwendende Kraft in einer Höhe gewählt wird, die innerhalb einer Kalibrierzeit eine muskuläre Ermüdung hervorruft, ohne den analysierten Muskel in die Nähe seiner Leistungsgrenze zu treiben. So sollte innerhalb einer akzeptablen Kalibrierzeit eine deutliche muskuläre Ermüdung hervorgerufen werden. Auf diese Weise kann die zweite Kalibrierung in relativ kurzer Zeit mit gut verwertbaren Ergebnissen durchgeführt werden. In einer optimalen Variante der Erfindung liegt die vom Muskel aufgebrachte relative Muskelkraft zwischen etwa 20% und etwa 40% der maximalen willkürlichen Kontraktionskraft dieses Muskels. Hiermit lassen sich besonders gute Auswertungsergebnisse für die tatsächliche muskuläre Ermüdung des Muskels erzielen, ohne dass der Muskel zu stark beansprucht wird.

In einer speziellen Weiterbildung der vorliegenden Erfindung wird wenigstens ein weiterer elektromyographischer Kennwert ermittelt und in den funktionalen Zusammenhang zwischen der tatsächlichen Muskelermüdung und der Differenz (ΔEMG) einbezogen. Hierdurch kann eine weitere Verbesserung der Kraftunabhängigkeit der Ermüdungsbestimmung ermöglicht werden, wobei die vorgeschlagene Vorgehensweise insbesondere dann sinnvoll ist, wenn die Nichtlinearität der Änderung der verwendeten EMG-Kennwerte bei Ermüdung durch differente Kräfte sehr unterschiedlich ist.

Beispielsweise kann ein solcher weiterer elektromyographischer Kennwert ein bereits zur Ermittlung der Differenz ΔEMG verwendeter elektromyographischer Kennwert sein. Wichtig ist, dass der Kennwert im starken Maß von der relativen Muskelkraft abhängig ist. Beispielsweise kann hierbei nochmals die elektrische Aktivität verwendet werden. Nach der definierten Beanspruchung stehen somit Daten-Trios bzw. noch weitere Daten zur Approximation zur Verfügung, die beispielsweise aus der erwarteten (tatsächlichen) Ermüdung, der Differenz ΔEMG und der Elektrischen Aktivität bestehen.

In einem anderen Beispiel der vorliegenden Erfindung kann der weitere elektromyographische Kennwert ein nicht zur Ermittlung der Differenz ΔEMG verwendeter elektromyographischer Kennwert sein, der möglichst stark von der Variation der aufgewendeten Muskelkraft und möglichst wenig von der muskulären Ermüdung abhängig ist. Dieser Kennwert wird während der Kalibrierphase erfasst, in die approximierte Funktion zur Bestimmung der Ermüdung aus der Differenz ΔEMG einbezogen und bewirkt eine Verbesserung der Kraftunabhängigkeit der Ermüdungsbestimmung.

Gemäß einer besonders favorisierten Weiterbildung der vorliegenden Erfindung wird zur Ermittlung der tatsächlichen Muskelermüdung eine erste Muskelbeanspruchung mit einer Kalibrierung und danach wenigstens eine zweite Muskelbeanspruchung mit einer Kalibrierung durchgeführt, wobei bei der wenigstens einen zweiten Muskelbeanspruchung die vom Muskel aufzuwendende Kraft kleiner oder größer als bei der ersten Muskelbeanspruchung ist und die Relation der bei den wenigstens zwei Muskelbeanspruchungen aufgewendeten Kräfte zueinander bekannt ist, und es wird ein funktionaler Zusammenhang für eine tatsächliche Ermüdung in Abhängigkeit von der Differenz ΔEMG derart ausgewählt, dass er sowohl für die Differenz zwischen dem aktuell ermittelten elektromyographischen Kennwert und dem funktionalen Zusammenhang zwischen den wenigstens zwei elektromyographischen Kennwerten bei der ersten Muskelbeanspruchung als auch für die Differenz ΔEMG zwischen dem aktuell ermittelten Kennwert und dem funktionalen Zusammenhang zwischen den wenigstens zwei elektromyographischen Kennwerten bei der wenigstens einen zweiten Muskelbeanspruchung bzw. jeder weiteren Muskelbeanspruchung passend ist. Diese zusätzliche Kalibrierung (Ermüdungskalibrierung) ist vorzugsweise dann einzusetzen, wenn die relative Höhe der Muskelkraft nicht bekannt ist. Da jedoch bei dem Kalibrierschritt die Relation der bei den beiden Muskelbeanspruchungen aufgewendeten Kräfte zueinander bekannt ist, wobei die tatsächliche Höhe der Muskelkräfte nicht bekannt sein muss, kann anhand der gemessenen Werte dennoch hinreichend genau ein Wert für die muskuläre Ermüdung bestimmt werden.

In einer speziellen Ausführungsvariante der vorliegenden Erfindung wird die wenigstens eine zweite Kalibrierung nach einer vollständigen Erholung des beanspruchten Muskels durchgeführt. Es ist jedoch auch möglich, dass der Muskel noch nicht vollständig erholt ist, aber die vorhandene Ermüdung des Muskels zu Beginn der zweiten Muskelbeanspruchung bekannt ist. Somit kann die zweite Muskelbeanspruchung bei definierten Bedingungen gestartet werden, so dass eine genaue Ermittlung der tatsächlichen muskulären Ermüdung möglich ist.

Vorzugsweise wird die relative Muskelkraft bei der zweiten Muskelbeanspruchung im Vergleich zu der ersten Muskelbeanspruchung verdoppelt. Dies ermöglicht eine besonders einfache Auswertung der gemessenen Werte.

Ferner kann erfindungsgemäß eine relative Muskelkraft aus der Differenz ΔEMG und aus den bei wenigstens zwei unterschiedlichen Muskelbeanspruchungen ermittelten funktionalen Zusammenhänge zwischen den jeweils wenigstens zwei elektromyographischen Kennwerten ermittelt werden. Es reicht damit zur Bestimmung der relativen Muskelkraft aus, die Differenz ΔEMG und das Verhältnis der verwendeten Muskelbeanspruchungen zueinander zu kennen.

In einer weiteren, empfehlenswerten Weiterbildung der vorliegenden Erfindung wird eine muskuläre Beanspruchung ermittelt, indem aus der Differenz ΔEMG ein Zusammenhang zwischen den wenigstens zwei ermittelten elektromyographischen Kennwerten in einem Zustand ohne muskuläre Ermüdung ermittelt wird, aus welchem im statischen Lastfall eine relative Muskelbeanspruchung, beziehungsweise dann, wenn aus einem vorangegangenem Kalibrierschritt die Kraft in einem Zustand ohne muskuläre Ermüdung ermittelt wurde, eine absolute Muskelbeanspruchung ermittelt wird. Dabei ist unter der muskulären Beanspruchung die vom Muskel aufgebrachte Kraft, einschließlich der Art und der Nebenbedingungen, unter denen diese Kraft aufgebracht wird (d.h. statische/dynamische Kraft, Konzentrische/exzentrische/isometrische Kontraktion des Muskels) zu verstehen. Somit kann man zusätzlich zu der muskulären Ermüdung während der Messphase die aktuelle muskuläre Beanspruchung aus den EMG-Kennwerten zur Bestimmung der muskulären Ermüdung ermitteln.

In einer weiteren Variante der Erfindung wird eine relative muskuläre Beanspruchung aus dem zu Beginn der zweiten, zu einer definierten Muskelermüdung führenden Kalibrierung ermittelten elektromyographischen Kennwerten ermittelt, indem fehlende Beanspruchungswerte interpoliert und gegebenenfalls extrapoliert werden oder die Kennwerte mit einer geeigneten Funktion approximiert werden. So kann die relative Beanspruchung beziehungsweise die relative Muskelkraft im statischen Fall aus den in der Kalibrierung mit definierter Ermüdung zu Beginn der Beanspruchung am noch nicht ermüdeten Muskel gemessenen Kennwerten, wie beispielsweise der elektrischen Aktivität und der Medianfrequenz, bestimmt werden. Entsprechend ist eine quantitative Ermittlung der muskulären Beanspruchung allein aus den EMG-Kennwerten möglich.

Vorteilhafte Ausführungsformen der vorliegenden Erfindung, deren Funktion und Vorteile werden im Folgenden anhand der Figuren der Zeichnung näher erläutert, wobei

Figur 1 schematisch an einem Beispiel eine typische Messanordnung zur Erfassung und Digitalisierung elektromyographischer Signale zeigt;

Figur 2 schematisch anhand eines Beispiels die maximal erreichbare muskuläre Ermüdung in Abhängigkeit von der muskulären Beanspruchung zeigt;

Figur 3 ein Beispiel einer gemessenen Kurve, die die Elektrische Aktivität in Abhängigkeit von der Medianfrequenz darstellt, und ihre Approximation durch ein Polynom zweiter Ordnung zeigt;

Figur 4 schematisch eine Verschiebung eines Datums aus elektromyographischen Messwerten, wie der Elektrischen Aktivität und der Medianfrequenz, bei ermüdender Muskulatur und die Bestimmung eines neuen Kennwertes für die Differenz $\Delta$EMG zeigt;

Figur 5 schematisch eine resultierende Kurvenschar bei variierender Muskelkraft und muskulärer Ermüdung mit beispielsweise verwendeten EMG-Kennwerten wie der Elektrischen Aktivität und der Medianfrequenz zeigt;

Figur 6 schematisch Beispiele für zwei $\Delta$EMG-Kurven zeigt, die aus Werten der Elektrischen Aktivität und der Medianfrequenz zweier Kalibrierabschnitte mit differenter Muskelkraft resultieren;

Figur 7 Beispiele für zwei tatsächliche Ermüdungskurven zeigt, die aus Erholungszeitkurven von *Rohmert* errechnet und normiert wurden; und

Figur 8 ein Beispiel für die Überlagerung der gemessenen Ermüdungskurven (hier bei statischen Muskelkräften bekannter Kraftrelation) mit verschiedenen Soll-Ermüdungskurven von Kräften gleicher Relation zeigt.

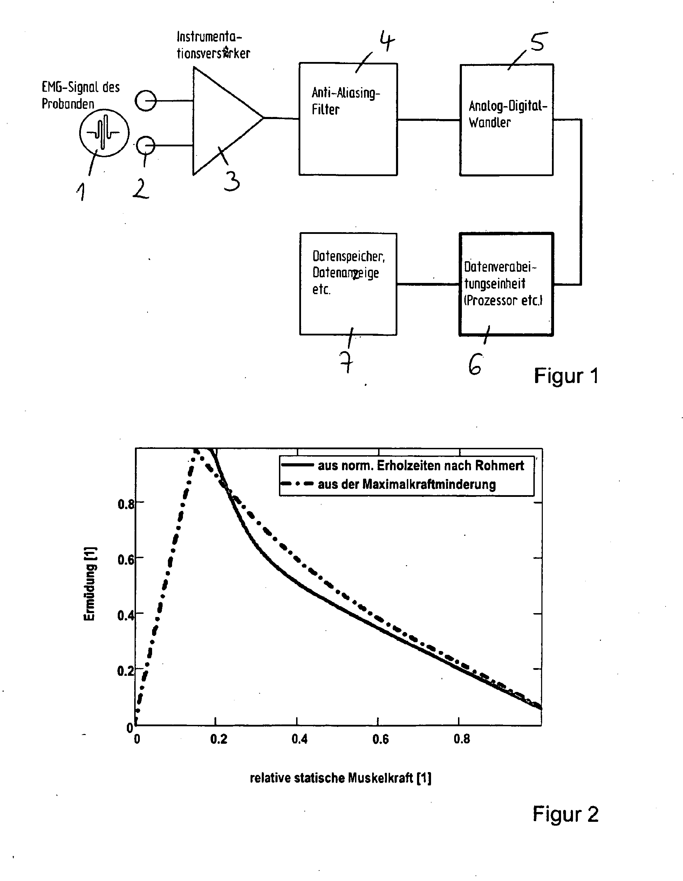

Figur 1 zeigt schematisch eine Variante einer typischen Messanordnung zur Erfassung und Digitalisierung elektromyographischer Signale, welche in dem Verfahren der vorliegenden Erfindung verwendet werden, um eine muskuläre Ermüdung quantitativ bestimmen zu können. Hierzu werden in üblicher Weise elektromyographische Rohsignale 1, vorzugsweise oberflächenmyographische Rohsignale, am Muskel mittels Elektroden 2 abgegriffen, über Instrumentationsverstärker 3, Filter 4 und Analog-Digital-Wandler 5 in mittels Computer bzw. Datenverarbeitungseinheit 6 verarbeitbare EMG-Signale gewandelt und in einer Speichereinheit 7 gespeichert. In der in Figur 1 dargestellten Anordnung wird die Datenverarbeitungseinheit 6 zur Ausführung aller Berechnungen verwendet. Alternativ ist eine Aufgabenverteilung auf mehrere Datenverarbeitungseinheiten möglich.

Die digitalisierten EMG-Rohsignale werden beispielsweise mittels Fourier-Transformation vom Zeit- in den Frequenzbereich transformiert. Aus dem EMG-Rohsignal und den Frequenzspektren werden in bekannter, in der Wissenschaft üblicher Weise mindestens zwei Kennwerte errechnet, die sowohl von der Ermüdung als auch von der vom analysierten Muskel aufgebrachten Kraft abhängen. Jeweils mindestens zwei Kenngrößen werden, wie weiter unten beschrieben, gemeinsam zu jeweils einer neuen Kenngröße, die nachfolgend Differenz oder EMG-Veränderung $\Delta$EMG genannt wird, umgewandelt.

Von Bedeutung für das Verfahren ist, dass die beiden zusammen verwendeten EMG-Kennwerte

a) sich gleichsinnig bei variierender Kraft und gegensinnig bei variierender Ermüdung oder

b) sich gegensinnig bei variierender Kraft und gleichsinnig bei variierender Ermüdung

verhalten.

In einer beispielhaft dargestellten Variante werden die bei EMG-Messungen häufig verwendeten Kennwerte, wie die Elektrische Aktivität EA (meist als Effektivwert, root mean square, rms) und die aus dem Frequenzspektrum ermittelte Medianfrequenz MF, verwendet. Sie verhalten sich bei Kraftvariation gleichsinnig, das heißt, beide Kenngrößen werden bei größer werdender Kraft ebenfalls größer und bei Ermüdungsvariation gegensinnig, das heißt, die Elektrische Aktivität nimmt mit zunehmender muskulärer Ermüdung ebenfalls zu und die Medianfrequenz nimmt dagegen mit zunehmender muskulärer Ermüdung ab.

Als objektives, quantitatives und vor allem universell vergleichbares Maß für die metabolisch muskuläre Ermüdung können zwei Größen herangezogen werden:

- der Betrag der Verringerung der maximal möglichen willkürlichen Kontraktionskraft eines Muskels, für die Anwendung im hier beschriebenen Verfahren auf 1 bzw. 100% normiert, oder
- die nach einer muskulären Ermüdung bis zur vollständigen Erholung erforderliche Erholzeit, für die Anwendung im hier beschriebenen Verfahren ebenfalls auf 1 bzw. 100% normiert.

Beide Maße beschreiben den Sachverhalt der muskulären Ermüdung objektiv, quantitativ und zwischen verschiedenen Muskeln und Individuen vergleichbar. Das Maximum der muskulären Ermüdung (1 bzw. 100%) wird bei muskulärer Beanspruchung in Höhe der Dauerleistungsgrenze (ca. 11-8% der maximal möglichen willkürlichen Kontraktionskraft) bei hinreichend langer Beanspruchungsdauer (theoretisch unendliche Dauer, praktisch etwa 20 bis 60 min) erreicht. Mit höheren oder niedrigeren Muskelkräften kann das Ermüdungsmaximum nicht erreicht werden.

Figur 2 verdeutlicht diesen Zusammenhang, indem schematisch anhand eines Beispiels die maximal erreichbare muskuläre Ermüdung in Abhängigkeit von der muskulären Beanspruchung gezeigt ist. Bei Muskelkräften oberhalb der Dauerleistungsgrenze ist die Beanspruchungsdauer außerdem physiologisch begrenzt. Die Beanspruchungshöhe wird vor allen durch die im Mittel eingesetzte Muskelkraft, aber auch durch das Verhältnis der statischen und dynamischen Anteile, die Periodendauer und das Tastverhältnis bei dynamischen Beanspruchungen und verschiedenen Ausführungsbedingungen wie konzentrisch-exzentrischer oder isometrischer Beanspruchung usw. bestimmt.

Die nach einer muskulären Ermüdung verbliebene maximal mögliche willkürliche Kontraktionskraft (maximal voluntary contraction, MVC) nimmt mit zunehmender muskulärer Ermüdung stetig ab. Die erforderliche Erholzeit nach einer muskulären Ermüdung nimmt mit der Höhe dieser Ermüdung ständig zu. Werden beide Werte auf 1 bzw. 100% normiert, führen sie zu ähnlichen Kurvenverläufen und gleichen Endwerten für die Ermüdung. Die normierte Verringerung der maximalen willkürlichen Kontraktionskraft ist das grundsätzlich exaktere Ermüdungsmaß, wobei sie bisher nur in relativ wenigen wissenschaftlichen Studien bestimmt wurde und deshalb eine deutlich kleinere Datenbasis zur Normierung zur Verfügung steht. Die normierte Erholzeit wird aus der, als "Erholzeitzuschlag" in wissenschaftlichen Untersuchungen erstmals von *Rohmert* definierten und später von vielen anderen Autoren bestätigten Zeitspanne zur vollständigen Erholung der Muskulatur nach verschieden hohen muskulären Ermüdungen abgeleitet. Sie liegt zur Zeit allerdings nur für muskuläre Beanspruchungen oberhalb der Dauerleistungsgrenze vor.

Beide Maße gelten bei einer bekannten Beanspruchungshöhe und einer bekannten Beanspruchungsdauer als "tatsächliche muskuläre Ermüdung" oder "erwartete muskuläre Ermüdung". Das in der vorliegenden Patentanmeldung beschriebene Verfahren ermöglicht u.a. eine Bestimmung der tatsächlichen oder erwarteten muskulären Ermüdung ausschließlich anhand von elektromyographischen Messwerten, d.h., ohne Kenntnis der aufgewandten Muskelkraft, der Nebenbedingungen; wie der konzentrischen/exzentrischen Beanspruchung, der dynamischen/statischen Anteile usw. und der Beanspruchungsdauer.

Das erfindungsgemäße Verfahren besteht aus mindestens einer Kalibrierphase und einer oder mehreren nachfolgenden Messphasen. Die Positionen der zur Signalableitung verwendeten EMG-Elektroden sollte zwischen Kalibrier- und Messphase(n) nicht verändert werden, da dies zu erheblichen Messfehlern führen kann.

In einer ersten Kalibrierphase erfolgt eine Ermittlung von wenigstens zwei elektromyographischen Kennwerten, welche sich gleichsinnig bei variierender Kraft und gegensinnig bei variierender muskulärer Ermüdung oder gegensinnig bei variierender Kraft und gleichsinnig bei variierender muskulärer Ermüdung verhalten, wobei eine Kraftvariation in einem Zustand vorgenommen wird, in dem der untersuchte Muskel im Wesentlichen nicht ermüdet ist.

Jeder zu analysierende Muskel muss zu Beginn der ersten Kalibrierung vollständig erholt sein oder die vorhandene muskuläre Ermüdung muss quantitativ bekannt sein. Innerhalb einer kurzen Zeitspanne, beispielsweise von etwa 5 Sekunden, wird jeder Muskel gleichzeitig oder nacheinander mit einer von Null beginnenden, stetig zunehmenden Kraft beansprucht. Die während der ersten Kalibrierung maximal vom Muskel aufzubringende Kraft sollte dabei mindestens die Höhe der höchsten in den anschließenden Messungen auftretenden Kräfte erreichen, um Ungenauigkeiten durch Extrapolationen möglichst zu vermeiden.

Während dieser Zeitspanne werden die mindestens zwei der oben genannten EMG-Kennwerte, die von der Muskelkraft abhängen, kontinuierlich oder wenigstens bei mehreren unterschiedlichen Größen der Kraft gemessen bzw. aus den Messdaten errechnet.

In einem Beispiel ergibt sich sowohl für den gewählten Frequenzkennwert "Medianfrequenz MF" als auch für den gewählten Amplitudenkennwert "Elektrische Aktivität EA" eine Zunahme bei steigender Kraftanforderung an den Muskel. Alle gemessenen bzw. berechneten Kennwerte $KW_x$ lassen sich als Funktion der vergangenen Kalibrierzeit $t_{kal}$ darstellen:

$$KW_X = f(t_{Kal}).$$

In dem gewählten Beispiel gilt

$$MF = f(t_{Kal}) \text{ und } EA = f(t_{Kal}).$$

Wird in den nachfolgenden Messungen eine Ermittlung der vom Muskel aufgewandten Kraft aus den EMG-Daten gewünscht, muss in der ersten Kalibrierphase neben den mindestens zwei EMG-Kennwerten auch die vom Muskel aufgebrachte relative Kraft (relativ zu seiner MVC) kontinuierlich oder wenigstens bei mehreren unterschiedlichen Größen der Kraft gemessen werden. Alle Kennwerte lassen sich in diesem fall auch als Funktion der relativen Muskelkraft $F_{rel}$ darstellen:

$$KW_X = f(F_{rel}).$$

Im gewählten Beispiel gilt dann zusätzlich:

$$MF = f(F_{rel}) \text{ und } EA = f(F_{rel}).$$

Soll nur die muskuläre Ermüdung und nicht die Muskelkraft aus den EMG-Daten bestimmt werden, muss die Kraftkurve bzw. einzelne Punkte davon während der Kalibrierung nicht aufgezeichnet werden.

Die erste Kalibrierphase sollte für jeden Muskel entweder in einer relativ kurzen Zeitspanne, oder in mehreren Kraftabschnitten mit dazwischen liegenden ausreichenden Pausen ausgeführt werden, da muskuläre Ermüdungserscheinungen während der Kalibrierphase zu erhöhten Messfehlern führen.

Ist mindestens einer der aus dem EMG ermittelten Kennwerte nicht eineindeutig mit der Muskelkraft verknüpft, muss ein weiterer EMG-Kennwert als Entscheidungskriterium hinzugezogen werden, um an jedem Punkt der Kurve eine eindeutige Zuordnung von Kennwert zu Kraft und Kraft zu Kennwert zu ermöglichen. Die gemessenen bzw. berechneten Kennwertverläufe werden miteinander so verknüpft, dass ein Kennwert als Funktion eines anderen Kennwertes beschrieben wird, d.h. $KW_{X1} = f(KW_{X2})$. Das ist problemlos möglich, da alle Kennwerte, wie oben beschrieben, als Funktion der Zeit und/oder als Funktion der Muskelkraft zur Verfügung stehen.

Die einzelnen Werte der Kennwertkurve $KW_X = f1(t_{Kal})$, also $KW_{X1..Xn}$ und $KW_Y = f2(t_{Kal})$, d.h. $KW_{Y1..Yn}$ werden gegeneinander aufgetragen und ergeben die neue Funktion $KW_Y = f3(KW_X)$. Wenn mehr als zwei EMG-Kennwerte verwendet werden, ergibt sich folgende Abhängigkeit: $KW_Y = f3(KW_{X1}, KW_{X2,...,} KW_{Xn})$. Im gewählten Beispiel wird aus $EA = f(t_{Kal})$ und $MF = (t_{Kal})$ ebenso wird aus $EA = f(F_{rel})$ und $MF = (F_{rel})$ letztlich $EA = f(MF)$ oder $MF = f(EA)$. Im Beispiel wird $EA = f(MF)$ gewählt.

Grundsätzlich kann die weitere Datenverarbeitung anhand der gemessenen bzw. berechneten Einzelwerte der Kennwerte mit einer gegebenenfalls erforderlichen Interpolation der Kennwertkurven zwischen den Werten erfolgen. Vorteilhafter ist jedoch die Approximation durch mathematische Funktionen, da dies die weiteren Berechnungen in der Regel deutlich vereinfacht. In einem Beispiel wird eine Approximation mittels Polynom 2. Ordnung gewählt, da diese die meisten Kennwertkurven recht gut abbildet und auch in kleineren Messgeräten bzw. von deren Prozessor noch einfach zu berechnen ist. Für das gewählte Beispiel wird dementsprechend aus den während der Kalibrierung mit variierender Kraft gemessenen bzw. berechneten EA- und MF-Werten die Approximation mit den Polynomkoeffizienten $kA_{MF}$, $kB_{MF}$ und $kC_{MF}$ gewählt:

$$MF = kA_{MF} \cdot EA^2 + kB_{MF} \cdot EA + kC_{MF}$$

Wurde während der Kalibrierphase auch die Muskelkraft gemessen, stehen zusätzlich die Zusammenhänge

$$EA = kA_{EA.F} \cdot Fr^2 + kB_{EA.F} \cdot Fr$$

und

$$MF = kA_{MF.F} \cdot Fr^2 + kB_{MF.F} \cdot Fr + kC_{MF.F}$$

zur weiteren Verwendung zur Verfügung.

Somit erfolgt erfindungsgemäß eine Ermittlung eines funktionalen Zusammenhangs zwischen den wenigstens zwei elektromyographischen Kennwerten.

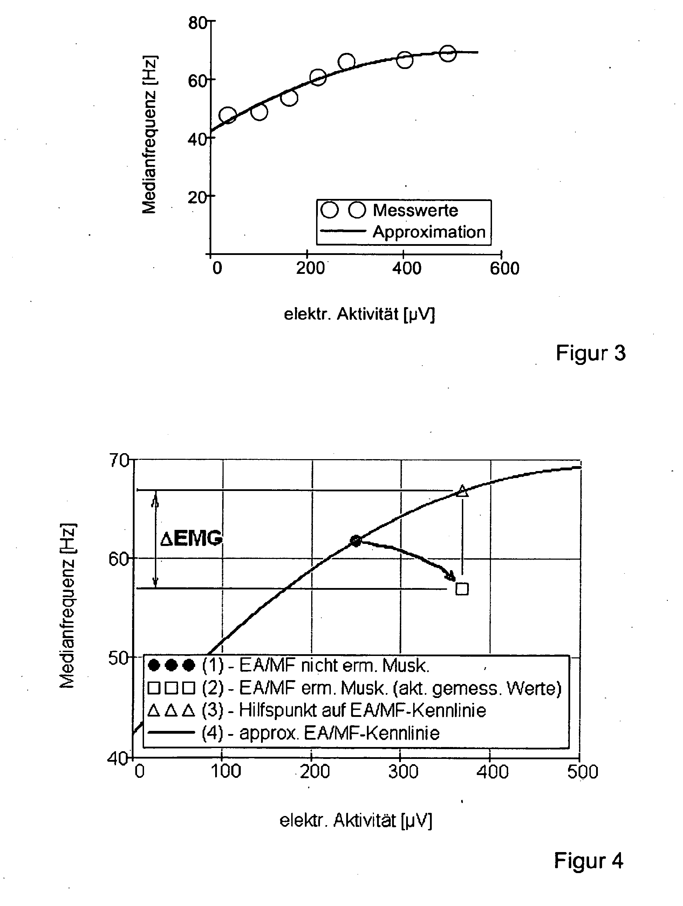

Figur 3 zeigt schematisch ein Beispiel einer gemessenen Kurve, die die Medianfrequenz in Abhängigkeit von der Elektrischen Aktivität darstellt, und ihre Approximation durch ein Polynom zweiter Ordnung.

Weiterhin wird erfindungsgemäß in einer sich an die Kalibrierung anschließenden Mess- und Auswertephase eine Differenz ∆EMG zwischen wenigstens einem aktuell ermittelten elektromyographischen Kennwert und dem funktionalen Zusammenhang zwischen den wenigstens zwei in der Kalibrierphase ermittelten elektromyographischen Kennwerten bestimmt. Die neue EMG-Kenngröße ∆EMG bzw. die EMG-Veränderung stellt ein vorläufiges Maß für die muskuläre Ermüdung dar.

Wie bereits erwähnt, werden jeweils mindestens zwei Kennwerte gemeinsam zu jeweils einer neuen Kenngröße, der EMG-Veränderung bzw. der Differenz ∆EMG umgewandelt. Werden mehr als zwei Kennwerte aus dem EMG verwendet, entstehen mehrere neue Kenngrößen EMG-Veränderung$_{1..n}$.

Die Bestimmung der neuen Kenngröße ∆EMG sei zunächst graphisch an dem in Figur 4 dargestellten Diagramm der Kurve $KW_Y = f(KW_X)$ dargestellt: Für die beispielhaft verwendeten EMG-Kenngrößen EA und MF wurde in der ersten Kalibrierung (Kraftvariation ohne Ermüdung) der Zusammenhang MF= f(EA) ermittelt und beispielhaft mit einem Polynom 2. Ordnung als mathematische Funktion

$$MF = kA_{MF} \cdot EA^2 + kB_{MF} \cdot EA + kC_{MF}$$

approximiert.

Sofern der analysierte Muskel nicht ermüdet und die Position der EMG-Elektroden 2 gegenüber dem Muskel nicht verändert wird, bewegen sich die gemessenen bzw. aus den EMG-Messwerten berechneten EA- und MF-Werte bei variierender Kraft stets auf der kalibrierten Kurve MF=f(EA), (Figur 4, (4)). Ermüdet der analysierte Muskel, verändern sich EA und MF in bekannter Weise: Die Elektrische Aktivität wird größer, die Medianfrequenz nimmt dagegen ab. Ein Datum, dass bei vollkommen erholtem Muskel auf der EA-MF-Kurve liegt (Figur 4, (1)) und durch eine bestimmte relative Muskelkraft bestimmt ist, wandert nach rechts unten, hin zu höheren EA- und niedrigeren MF-Werten. Es entspricht den am ermüdeten Muskel gemessenen bzw. berechneten EA- und MF-Werten (Figur 4, (2)). Der Kennwert ∆EMG für das Kennwertepaar EA/MF ist der Betrag der Differenz zwischen dem MF-Wert dieses Punktes und dem MF-Wert des Hilfspunktes (Figur 4, (3)) auf der kalibrierten EA-MF-Kurve, der senkrecht über dem gemessenen Punkt (Figur 4, (2)) liegt. Dieselbe Vorgehensweise ergibt sich, wenn statt des senkrechten Abstandes ein waagerechter Abstand zwischen ∆EMG und der kalibrierten Kennlinie verwendet wird.

Das gewählte Beispiel mit Approximation der EA-MF-Kurve des nicht ermüdeten Muskels mittels Polynom 2. Ordnung ergibt:

$$\Delta EMG(E) = \left| MF_{ist} - \left( kA_{MF} \cdot EA^2 + kB_{MF} \cdot EA + kC_{MF} \right) \right|$$

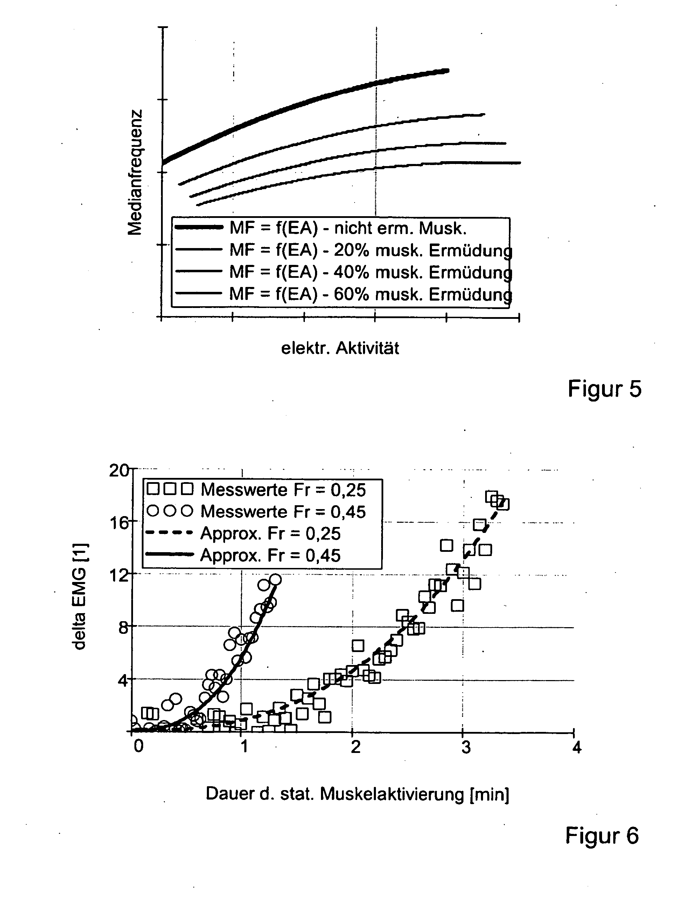

Bei einer angenommen konstanten muskulären Ermüdung, die größer als Null ist, und variierender Kraft entsteht aus den Koordinaten der gewählten Kennwerte eine Linie, die gegenüber der aus der Kalibrierung resultierenden Linie verschoben, dieser jedoch ähnlich ist. Im gewählten Beispiel der EA-MF-Kennlinie entsteht aus verschieden hohen diskreten Ermüdungen bei variierender Kraft eine Kurvenschar rechts unterhalb der am völlig erholten Muskel während der ersten Kalibrierung aufgenommenen Kennlinie. Entsprechend zeigt Figur 5 eine resultierende Kurvenschar bei variierender Muskelkraft und muskulärer Ermüdung mit den beispielhaft gewählten EMG-Kennwerten EA und MF.

Die so ermittelte Kenngröße ΔEMG ist ein von der angewandten Muskelkraft noch nicht vollständig, aber bereits weitgehend unabhängiges qualitatives bzw. semiquantitatives Maß für die Ermüdung des analysierten Muskels. Die Kenngröße ΔEMG steigt monoton mit zunehmender Ermüdung und sinkt mit abnehmender Ermüdung.

In einer Erweiterung des Verfahrens werden in ähnlicher Weise mehr als zwei EMG-Kennwerte zu einer neuen Kenngröße ΔEMG umgewandelt. Dabei wird der in der ersten Kalibrierphase, Teil 1, ermittelte Zusammenhang

$$KW1=f(KW2, KW3, ...KWn)$$

genutzt. Die Kenngröße ΔEMG wird wie im vorstehend beschrieben Verfahren berechnet. Bei geeigneter Wahl der EMG-Kennwerte wird die Unabhängigkeit von der Kraftvariation verbessert und eine insgesamt höhere Genauigkeit erreicht.

In einer vorteilhaften Ausgestaltung der Erfindung wird in einer zweiten Kalibrierung ein funktionaler Zusammenhang zwischen den wenigstens zwei elektromyographischen Kennwerten bei definierter Muskelermüdung ermittelt wird, fortlaufend oder in diskreten zeitlichen Abständen die Differenz ΔEMG zwischen einem aktuell ermittelten elektromyographischen Kennwert und dem funktionalen Zusammenhang bestimmt, aus der Höhe der relativen Muskelkraft bei der Kalibrierbeanspruchung und der Dauer der Kalibrierbeanspruchung eine tatsächliche Muskelermüdung ermittelt, und ein funktionaler Zusammenhang zwischen der tatsächlichen Muskelermüdung und der Differenz ΔEMG ermittelt. Das heißt, bei der zweiten Kalibrierung wird eine definierte Ermüdung der untersuchten Muskeln durchgeführt.

Der bei der ersten Kalibrierung ermittelte Kennwert ΔEMG bzw. die ermittelten Kennwerte $\Delta EMG_{1..n}$, falls mehr als zwei EMG-Kennwerte als Ursprung verwendet wurden, stellt ein semiquantitatives Maß für die metabolisch muskuläre Ermüdung dar. Für die Transformation in exakte Ermüdungswerte in Bereich 0 bis 1 bzw. 0 bis 100% ist somit eine weitere, zweite Kalibrierung mit "definiert muskulärer Ermüdung" erforderlich. Bei geringen Anforderungen an die Messgenauigkeit kann dieser Schritt entfallen. Stattdessen werden dann "durchschnittliche Transformationsparameter" verwendet, sofern diese für den analysierten Muskel, die Elektrodenposition und die Art und Größe der Elektroden bekannt sind.

Die zusätzliche, zweite Kalibrierung erfüllt noch eine weitere Aufgabe: Der berechnete Kennwert ΔEMG ist bei angenommen konstanter muskulärer Ermüdung nicht unter allen Umständen vollständig unabhängig von der Variation der Muskelkraft. Ursache dafür ist die Nichtlinearität, vor allem die im Vergleich der mindestens zwei verwendeten EMG-Kennwerte unterschiedliche Nichtlinearität der Änderung der EMG-Kennwerte bei Ermüdung durch differente Kräfte. Die zusätzliche Kalibrierung kann die Kraftunabhängigkeit der Ermüdungsmessung in Abhängigkeit vom bemessenen Muskel und einer Reihe weiterer Faktoren verbessern.

Bei der zweiten Kalibrierung müssen die zu analysierenden Muskeln wiederum vollständig erholt sein oder die ihre vorhandene Vorermüdung muss in ihrer Quantität bekannt sein. Alle zu analysierenden Muskeln werden gleichzeitig oder nacheinander statisch oder dynamisch mit einer Kraft konstanter Höhe für eine definierte Zeit ermüdet, wobei wiederum die ausgewählten EMG-Kennwerte kontinuierlich oder in definierten zeitlichen Abständen gemessen bzw. aus den Messdaten berechnet und gegebenenfalls gespeichert werden.

Die vom Muskel aufzuwendende Kraft muss nicht zwingend bekannt sein, sollte vorzugsweise aber in einer Höhe gewählt werden, die innerhalb einer akzeptablen Kalibrierzeit eine deutliche muskuläre Ermüdung erwarten lässt, ohne den analysierten Muskel in die Nähe seiner Leistungsgrenze zu treiben. Optimal sind Werte für die "relative Muskelkraft", das heißt, den Anteil an der maximalen willkürlichen Kontraktionskraft dieses Muskels, zwischen etwa 20% und etwa 40%. Aus den während dieser Kalibrierbeanspruchung gemessenen EMG-Kennwerten wird mindestens eine Kenngröße ΔEMG nach oben beschriebenem Verfahren fortlaufend oder in diskreten zeitlichen Abständen berechnet.

Ist die Höhe der relativen Muskelkraft dieser Kalibrierbeanspruchung bekannt, ist damit auch die zu erwartende Ermüdungskurve, das heißt, die stets nichtlineare Zunahme der Ermüdung mit der Beanspruchungsdauer bekannt. Somit kann der Zusammenhang zwischen zu erwartender (tatsächlicher) Ermüdung und dem oder den ermittelten ΔEMG-Wert(en) hergestellt, ΔEMG also in eine "echte Ermüdung" transformiert werden. Vorzugsweise wird dieser Zusammenhang wieder mittels einer mathematischen Funktion approximiert. Aus den Wertepaaren von erwarteter, das heißt, bekannter Ermüdung und ΔEMG können beispielsweise die Koeffizienten eines Polynoms zweiter Ordnung, $kA_E$ und $kB_E$, zur Bestimmung der Ermüdung E aus ΔEMG ermittelt werden:

$$E = kA_E \cdot \Delta EMG(E)^2 + kB_E \cdot \Delta EMG(E)$$

Wird die weiter oben beispielhaft beschriebene Bestimmung von $\Delta EMG$

$$\Delta EMG(E) = \left| MF_{ist} - \left( kA_{MF} \cdot EA^2 + kB_{MF} \cdot EA + kC_{MF} \right) \right|$$

in die Formel eingesetzt, entsteht zur Bestimmung der Ermüdung E folgende Gleichung

$$E = kA_E \left[ \left| MF_{ist} - \left( kA_{MF} \cdot EA^2 + kB_{MF} \cdot EA + kC_{MF} \right) \right| \right]^2 + kB_E \left[ \left| MF_{ist} - \left( kA_{MF} \cdot EA^2 + kB_{MF} \cdot EA + kC_{MF} \right) \right| \right] ,$$

deren Koeffizienten aus den beiden Kalibrierphasen ermittelt wurden.

Eine weitere Verbesserung der Kraftunabhängigkeit der Ermüdungsbestimmung ist möglich und insbesondere dann sinnvoll, wenn die weiter oben beschriebene Nichtlinearität der Änderung der verwendeten EMG-Kennwerte bei Ermüdung durch differente Kräfte sehr unterschiedlich ist. Zu diesem Zweck wird in der vorstehend beschriebenen Kalibrierphase ein weiterer EMG-Kennwert erfasst und in die approximierte Funktion zur Bestimmung der Ermüdung aus $\Delta EMG$ einbezogen. Optimal ist dafür ein beliebiger aus dem EMG ermittelter Parameter geeignet, der nicht für die Ermittlung von $\Delta EMG$ verwendet wurde, in starkem Maße von der Variation der aufgewendeten Muskelkraft und in möglichst geringem Maße von der muskulären Ermüdung abhängig ist. Im einfachsten Fall kann dies aber auch ein bereits verwendeter EMG-Kennwert sein. Wichtig ist, dass der Kennwert in starkem Maße von der relativen Muskelkraft abhängig ist. Beispielhaft sei hier noch einmal die Elektrische Aktivität EA verwendet. Nach der definierten Beanspruchung stehen Daten-Trios zur Approximation zur Verfügung, die im Beispiel aus erwarteter (tatsächlicher) Ermüdung, der Differenz $\Delta EMG$ und der Elektrischen Aktivität EA bestehen. Zur Bestimmung der Ermüdung E kann die obige Gleichung dann beispielsweise wie folgt erweitert werden:

$$E = kA_E \left[ \left| MF_{ist} - \left( kA_{MF} \cdot EA^2 + kB_{MF} \cdot EA + kC_{MF} \right) \right| \right]^2 + kB_E \left[ \left| MF_{ist} - \left( kA_{MF} \cdot EA^2 + kB_{MF} \cdot EA + kC_{MF} \right) \right| \right] + kC_E \cdot EA^2 + kD_E \cdot EA$$

Ist in der vorstehend beschriebenen, zweiten Kalibrierphase (Ermüdungskalibrierung) die relative Höhe der Muskelkraft nicht bekannt, ist ein zusätzlicher Kalibrierabschnitt in dieser Phase erforderlich: Direkt im Anschluss an die vorgenannte Beanspruchung oder, vorzugsweise, nach einer hinreichend langen Pause, die eine vollständige Erholung des Muskels ermöglicht, wird diese Kalibrierung mindestens ein weiteres Mal durchgeführt. Sämtliche Ausführungsbedingungen (z.B. Haltung, statische/dynamische Beanspruchungsanteile usw.) dürfen dabei nicht verändert werden.

Die vom Muskel aufzuwendende Kraft muss kleiner oder größer als die im vorangegangenen Kalibrierabschnitt aufgewandte Kraft sein. Die Relation der beiden Muskelkräfte zueinander muss zwingend bekannt sein. Die tatsächliche Höhe der Muskelkräfte muss nicht bekannt sein. Optimal ist eine Verdopplung der relativen Muskelkraft, sofern im vorhergehenden Abschnitt mit etwa 20% bis etwa 40% relativer Muskelkraft kalibriert wurde.

Es entsteht eine weitere nichtlineare Kurve des Kennwerts $\Delta EMG$. Gegenüber der $\Delta EMG$-Kurve aus dem vorhergehenden Kalibrierabschnitt ist die Änderung des Anstiegs dieser neuen Kurve, das heißt, ihre Krümmung bzw. ihre zweite Ableitung nach der Zeit, different gegenüber der ersten. Ist beispielsweise die relative Muskelkraft gegenüber der vorangehenden Kalibrierung größer und wurden beide Kalibrierungen an einem völlig erholten Muskel durchgeführt, ist die Änderung des Anstiegs dieser zweiten Kurve an jedem, jeweils vom Beginn der Beanspruchung aus gemessenen Zeitpunkt größer als der der ersten Kurve. Dadurch ist die Form einer jeden solchen $\Delta EMG$-Kurve eindeutig bestimmt. Figur 6 verdeutlicht beispielhaft diesen Zusammenhang, indem hier Beispiele für zwei $\Delta EMG$-Kurven und ihrer Approximation gezeigt sind, die aus EA- und MF-Werten zweier Kalibrierabschnitte mit differenter Muskelkraft resultieren.

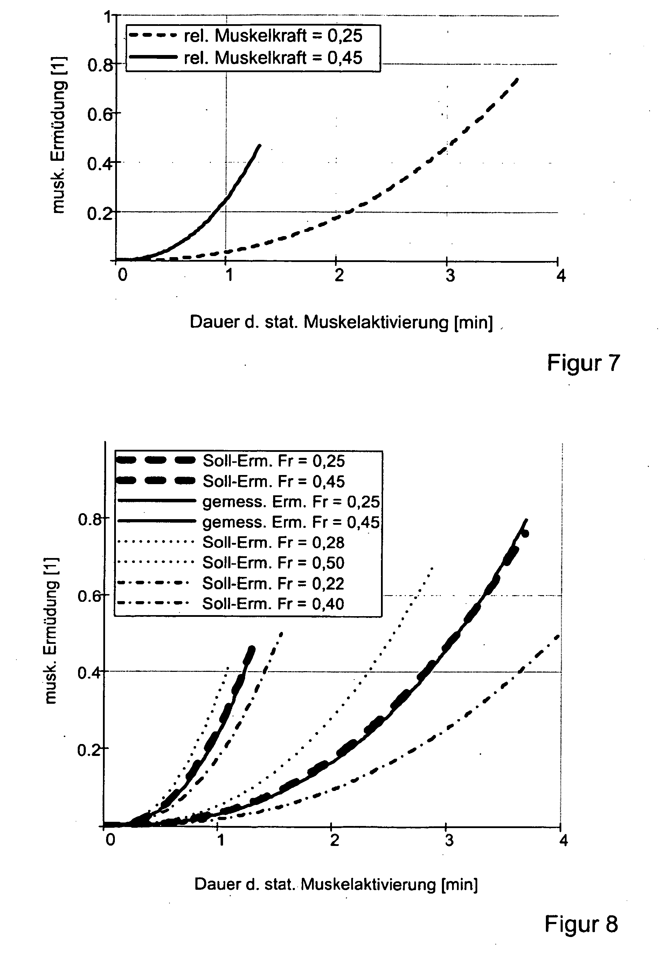

Ein ähnliches Verhalten zeigen auch die Kurven der tatsächlichen muskulären Ermüdung. Figur 7 zeigt eine Schar von Kurven tatsächlicher Ermüdung, wobei jede einzelne Kurve durch konstante muskuläre Beanspruchung mit fortschreitender Beanspruchungsdauer gekennzeichnet ist. Zwischen den Kurven wurde der ScharParameter "relative Muskelkraft" variiert. Der mit zunehmender relativer Muskelkraft schneller wachsende Anstieg der Kurven ist deutlich zu erkennen und führt zu einer charakteristischen und vor allem eindeutigen Form einer jeden Ermüdungskurve.

Um den oder die Kennwerte ΔEMG in "echte" Ermüdungswerte E zu transformieren, ist erfindungsgemäß, wie bereits oben beschrieben, eine Transferfunktion E = f(ΔEMG) erforderlich. Da das Verhältnis der vom Muskel aufgebrachten Kraft bzw. der muskulären Beanspruchung in den beiden Kalibrierabschnitten der Ermüdungskalibrierung bekannt ist bzw. zwingend bekannt sein muss, wird durch dieses Verhältnis auch das Verhältnis der beiden Scharparameter und damit eine Auswahl an Kurvenpaaren der muskulären Ermüdung bestimmt. War beispielsweise die muskuläre Beanspruchung im zweiten Kalibrierabschnitt doppelt so groß wie im ersten, sind dadurch Kurvenpaare mit eben diesem Verhältnis des Scharparameters "muskuläre Beanspruchung", z.B. 0,2/0,4 oder 0,25/0,5 oder 0,4/0,8 usw. definiert.

Die Transferfunktion E = F(ΔEMG) ist nun so zu wählen, dass sie für beide Ermüdungs-ΔEMG-Kurvenpaare möglichst gut passt, das heißt, die entstehenden Fehler minimiert sind. Aufgrund der nichtlinearen Eigenschaften der Ermüdungs- und ΔEMG-Kurven ist dies nur bei genau den zwei Scharparametern möglich, die den relativen muskulären Beanspruchungen (im statischen Falle vereinfacht: den relativen Muskelkräften) während der Kalibrierung entsprechen (Figur 8). Damit ist nicht nur die Transferfunktion zwischen ΔEMG und tatsächlicher Ermüdung gefunden, auch die beiden Kalibrierbeanspruchungen bzw. relativen Muskelkräfte, von denen bisher nur ihr Verhältnis bekannt war, sind exakt bestimmt. Werden mehr als zwei Ermüdungskalibrierabschnitte ausgeführt, vorzugsweise jeweils mit differenten Kräften bekannter Relation, kann die ΔEMG-Ermüdungs-Transferfunktion und damit auch die Höhe der Kräfte bzw. der Kalibrierbeanspruchungen genauer bestimmt werden.

Eine Vereinfachung lässt sich wie folgt realisieren: Sofern für den zu analysierenden Muskel die Transferfunktion ausgehend von ΔEMG zur Ermittlung der Ermüdung bereits bekannt ist, genügt die einmalige "Ermüdungs-Kalibrierung" mit einer unbekannten Muskelkraft. Der Kurvenverlauf der gemessenen muskulären Ermüdung, das heißt die aus den ΔEMG-Werten in Ermüdungswerte transformierte Kurve, lässt sich anhand ihrer Krümmung eindeutig einer bekannten Soll-Ermüdungskurve zuordnen. Damit ist auch in diesem Fall die aufgewandte relative Muskelkraft eindeutig bestimmt.

Die Kalibrierung ist damit abgeschlossen und die eigentliche Messung kann beginnen.

Hinsichtlich der Kalibrierbeanspruchung ist anzumerken, dass, wie bereits weiter oben beschrieben, die Beanspruchungshöhe neben der mittleren relativen Muskelkraft durch eine Reihe weiterer Einflussfaktoren wie dem Verhältnis der statischen und dynamischen Anteile, der Periodendauer und dem Tastverhältnis bei dynamischen Beanspruchungen und verschiedenen Ausführungsbedingungen wie konzentrischexzentrische oder isometrische Beanspruchung usw. bestimmt wird. Um während der Messphase eine möglichst große Genauigkeit der Ermüdungs- und ggf. der Muskelkraftmessung zu erreichen, sollten diese zusätzlichen Einflussfaktoren während der Kalibrierung in etwa den während der Messphase vorhandenen entsprechen. Werden während der Messphase Änderungen oder verschiedene Kombinationen dieser Einflussfaktoren auftreten, können für jede dieser Kombinationen die Transferfunktionen jeweils dafür zugeschnittener Kalibrierphasen oder "mittlere Transferfunktionen", die aus Kalibrierphasen mit einer geeigneten Mischung der Einflussfaktoren resultieren, verwendet werden.

Während der Messphase werden die EMG-Kennwerte kontinuierlich oder in zeitlichen Abständen gemessen, daraus der oder die ΔEMG-Kennwert(e) nach oben beschriebenem Verfahren und letztlich die muskuläre Ermüdung nach dem oben beschriebenen Verfahren berechnet.

Der so bestimmte quantitative Ermüdungswert entspricht der tatsächlichen muskulären Ermüdung in einem Bereich von 0 bis 1 bzw. 0 bis 100% und wird während der Messphase allein aus den aktuellen EMG-Kennwerten ohne Kenntnis der Beanspruchungshöhe, der bisherigen Beanspruchungsdauer und des bisherigen Ermüdungsverlaufs bestimmt. Er ist insbesondere kein iterativ berechneter Kennwert, weshalb Unterbrechungen der Messphase nicht zu Fehlern führen und ein Aufsummieren von Messfehlern nicht möglich ist.

Zusätzlich zur muskulären Ermüdung kann während der Messphase die aktuelle muskuläre Beanspruchung (im statischen Lastfall vereinfacht: die relative Muskelkraft) aus den EMG-Kennwerten und der gemessenen muskulären Ermüdung bestimmt werden: Die zu einem bestimmten Zeitpunkt gemessenen EMG-Kennwerte, im dargestellten Beispiel die Werte von EA und MF, sind die Koordinaten eines Datums im Diagramm $KW_Y = f(KW_X)$, im Beispiel das Diagramm MF = f(EA), das aufgrund der vorhandenen muskulären Ermüdung nicht auf der kalibrierten EA-MF-Kurve liegt (vgl. Figur 4). Betrag und Richtung der ermüdungsbedingten Verschiebung dieses Datums sind aufgrund des ermittelten Verlaufs der Änderung der EMG-Kennwerte bei definierter Ermüdung, im Beispiel die Änderung von EA und MF, bereits bekannt. Der Verlauf der EMG-Kennwertänderung wurde während der Kalibrierabschnitte "definierte Ermüdung" bestimmt und ggf. approximiert.

Da Betrag und Richtung dieser Verschiebung bekannt sind, kann der "Ursprungspunkt" auf der Kurve $KW_Y = f(KW_X)$, im Beispiel die Kurve MF = f(EA) ermittelt werden. Als "Ursprungspunkt" ist dabei das korrespondierende Datum auf der am völlig erholten Muskel kalibrierten Kurve bezeichnet. Ist der Punkt auf diese Kurve bekannt, ist auch die "relative Muskelkraft" (statischer Fall) respektive die "relative Beanspruchung" oder aber die absolute vom Muskel aufgebrachte Kraft bekannt, je nachdem, welche Kraft oder Beanspruchung im ersten Kalibrierabschnitt "Kraftvariation ohne Ermüdung" gemessen wurde. Wurden in diesem Abschnitt keine Kräfte gemessen, kann die "relative Beanspruchung" bzw. "relative Muskelkraft (statischer Fall)" auch aus den in der zweiten Kalibrierphase "Definierte Ermüdung" jeweils am Beginn der Beanspruchung, am noch nicht ermüdeten Muskel gemessenen EMG-Kennwerten, im Beispiel EA und MF, bestimmt werden. Da für die jeweiligen definierten Ermüdungskurven am Ende der Kalibrierphase die angewandte "relative Beanspruchung" bzw. die "relative Muskelkraft" bestimmt wurde, sind die mit den EMG-Kennwerten korrespondierenden Kräfte im Nachhinein bekannt. Da es sich hier um diskrete Werte für Kräfte/Beanspruchungen handelt, müssen in diesem Fall die fehlenden Beanspruchungswerte interpoliert und gegebenenfalls extrapoliert werden oder die Daten werden mit einer geeigneten Funktion approximiert.

In allen Fällen ist damit in der Messphase die quantitative Ermittlung der muskulären Beanspruchung allein aus den EMG-Kennwerten möglich.

**Patentansprüche**

1. Verfahren zum Bestimmen einer muskulären Ermüdung aus elektromyographischen Messwerten, wobei elektromyographische Rohsignale erfasst werden,
   aus den elektromyographischen Rohsignalen und/oder davon abgeleiteten Werten wenigstens zwei elektromyographische Kennwerte ermittelt werden, wobei sich wenigstens zwei der elektromyographischen Kennwerte gleichsinnig bei variierender Kraft und gegensinnig bei variierender Ermüdung oder gegensinnig bei variierender Kraft und gleichsinnig bei variierender Ermüdung verhalten,
   in wenigstens einer ersten Kalibrierung ein funktionaler Zusammenhang zwischen den wenigstens zwei elektromyographischen Kennwerten ermittelt wird, wobei bei dieser Kalibrierung im Wesentlichen keine Muskelermüdung auftritt, und
   in einer Mess- und Auswertephase eine Differenz ($\Delta$EMG) zwischen wenigstens einem aktuell ermittelten elektromyographischen Kennwert und dem funktionalen Zusammenhang zwischen den wenigstens zwei elektromyographischen Kennwerten bestimmt wird, und
   ein funktionaler Zusammenhang zwischen einer bekannten oder durch Kalibrierung ermittelten tatsächlichen muskulären Ermüdung und der Differenz ($\Delta$EMG) ermittelt wird und daraus ein Wert für die muskuläre Ermüdung ermittelt wird.

2. Verfahren nach Anspruch 1, **dadurch gekennzeichnet, dass** die elektromyographischen Rohsignale in ein Frequenzspektrum transformiert werden und aus dem Frequenzspektrum wenigstens ein elektromyographischer Kennwert ermittelt wird.

3. Verfahren nach einem der Ansprüche 1 oder 2, **dadurch gekennzeichnet, dass** bei der wenigstens einen ersten Kalibrierung eine Kraftvariation am Muskel durchgeführt wird und während der Kalibrierung wenigstens die zwei elektromyographischen Kennwerte und deren funktionaler Zusammenhang ermittelt werden, wobei bei der Kalibrierung im Wesentlichen keine Muskelermüdung auftritt.

4. Verfahren nach Anspruch 3, **dadurch gekennzeichnet, dass** bei der Kraftvariation am Muskel die zu analysierenden Muskeln gleichzeitig oder nacheinander mit einer stetig zunehmenden Kraft beansprucht werden und die wenigstens zwei elektromyographischen Kennwerte kontinuierlich oder wenigstens bei mehreren unterschiedlichen Kraftwerten ermittelt werden.

5. Verfahren nach einem der vorhergehenden Ansprüche, **dadurch gekennzeichnet, dass** bei der ersten Kalibrierung eine maximal vom Muskel aufzubringende Kraft so eingestellt wird, dass sie wenigstens der höchsten in einer anschließenden Messung auftretenden Kraft entspricht.

6. Verfahren nach einem der vorhergehenden Ansprüche, **dadurch gekennzeichnet, dass** bei der ersten Kalibrierung die vom Muskel relativ zu seiner maximalen willkürlichen Kontraktionskraft aufgebrachte Kraft kontinuierlich oder wenigstens bei mehreren unterschiedlichen Kraftwerten ermittelt wird.

7. Verfahren nach einem der vorhergehenden Ansprüche, **dadurch gekennzeichnet, dass** dann, wenn die wenigstens zwei elektromyographischen Kennwerte nicht eineindeutig mit der Muskelkraft verknüpft sind, wenigstens ein weiterer elektromyographischer Kennwert ermittelt und für eine eineindeutige Zuordnung zwischen Kennwert und Kraft verwendet wird.

8. Verfahren nach einem der vorhergehenden Ansprüche, **dadurch gekennzeichnet, dass** aus den elektromyographischen Rohsignalen wenigstens eine Elektrische Aktivität und aus dem Frequenzspektrum wenigstens eine Medianfrequenz oder ein Amplitudenverhältnis zweier oder mehrerer Frequenzbänder ermittelt wird.

9. Verfahren nach einem der vorhergehenden Ansprüche, **dadurch gekennzeichnet, dass** in einer zweiten Kalibrierung ein funktionaler Zusammenhang zwischen den wenigstens zwei elektromyographischen Kennwerten ermittelt wird, wobei der zu untersuchende Muskel definiert ermüdet wird, fortlaufend oder in diskreten zeitlichen Abständen eine Differenz (ΔEMG) zwischen einem aktuell ermittelten elektromyographischen Kennwert und dem funktionalen Zusammenhang bestimmt wird, aus der Höhe und Art der relativen Muskelkraft bei der Kalibrierbeanspruchung und der Dauer der Kalibrierbeanspruchung eine tatsächliche Muskelermüdung ermittelt wird, und ein funktionaler Zusammenhang zwischen der tatsächlichen Muskelermüdung und der Differenz (ΔEMG) ermittelt wird.

10. Verfahren nach Anspruch 9, **dadurch gekennzeichnet, dass** bei der zweiten Kalibrierung alle zu analysierenden Muskeln gleichzeitig oder nacheinander mit einer konstanten Kraft für eine definierte Zeit ermüdet werden, und während der Kalibrierung wenigstens die zwei elektromyographischen Kennwerte und deren funktionaler Zusammenhang in einem Zustand definierter Muskelermüdung ermittelt werden.

11. Verfahren nach Anspruch 9 oder 10, **dadurch gekennzeichnet, dass** die wenigstens zwei elektromyographischen Kennwerte kontinuierlich oder in definierten zeitlichen Abständen ermittelt werden.

12. Verfahren nach einem der Ansprüche 9 bis 11, **dadurch gekennzeichnet, dass** die vom Muskel aufzuwendende Kraft in einer Höhe gewählt wird, die innerhalb einer Kalibrierzeit eine muskuläre Ermüdung hervorruft, ohne den analysierten Muskel in die Nähe seiner Leistungsgrenze zu treiben.

13. Verfahren nach Anspruch 12, **dadurch gekennzeichnet, dass** die vom Muskel aufgebrachte relative Muskelkraft zwischen etwa 20% und etwa 40% der maximalen willkürlichen Kontraktionskraft dieses Muskels liegt.

14. Verfahren nach einem der Ansprüche 9 bis 13, **dadurch gekennzeichnet, dass** wenigstens ein weiterer elektromyographischer Kennwert ermittelt und in den funktionalen Zusammenhang zwischen der tatsächlichen muskulären Ermüdung und der Differenz (ΔEMG) einbezogen wird.

15. Verfahren nach Anspruch 14, **dadurch gekennzeichnet, dass** der weitere elektromyographische Kennwert ein bereits zur Ermittlung der Differenz (ΔEMG) verwendeter elektromyographischer Kennwert ist.

16. Verfahren nach Anspruch 14, **dadurch gekennzeichnet, dass** der weitere elektromyographische Kennwert ein nicht zur Ermittlung der Differenz (ΔEMG) verwendeter elektromyographischer Kennwert ist, der möglichst stark von der Variation der aufgewendeten Muskelkraft und möglichst wenig von der muskulären Ermüdung abhängig ist.

17. Verfahren nach einem der Ansprüche 9 bis 16, **dadurch gekennzeichnet, dass** zur Ermittlung der tatsächlichen muskulären Ermüdung eine erste Muskelbeanspruchung mit einer Kalibrierung und danach wenigstens eine zweite Muskelbeanspruchung mit einer Kalibrierung durchgeführt wird, wobei bei der wenigstens einen zweiten Muskelbeanspruchung die vom Muskel aufzuwendende Kraft kleiner oder größer als bei der ersten Muskelbeanspruchung ist und die Relation der bei den wenigstens zwei Muskelbeanspruchungen aufgewendeten Kräfte zueinander bekannt ist, und dass ein funktionaler Zusammenhang für eine tatsächliche muskuläre Ermüdung in Abhängigkeit von der Differenz (ΔEMG) derart ausgewählt wird, dass er sowohl für die Differenz zwischen dem aktuell ermittelten elektromyographischen Kennwert und dem funktionalen Zusammenhang zwischen den wenigstens zwei elektromyographischen Kennwerten bei der ersten Muskelbeanspruchung als auch für die Differenz zwischen dem aktuell ermittelten elektromyographischen Kennwert und dem funktionalen Zusammenhang zwischen den wenigstens zwei elektromyographischen Kennwerten bei der wenigstens einen zweiten Muskelbeanspruchung passend ist.

18. Verfahren nach Anspruch 17, **dadurch gekennzeichnet, dass** die wenigstens eine zweite Muskelbeanspruchung nach einer vollständigen Erholung des beanspruchten Muskels durchgeführt wird oder die muskuläre Ermüdung des zu untersuchenden Muskels vor Beginn der zweiten Muskelbeanspruchung bekannt ist.

19. Verfahren nach Anspruch 17 oder 18, **dadurch gekennzeichnet, dass** die relative Muskelkraft bei der zweiten Muskelbeanspruchung im Vergleich zu der ersten Muskelbeanspruchung verdoppelt wird.

20. Verfahren nach einem der Ansprüche 17 bis 19, **dadurch gekennzeichnet, dass** eine relative Muskelkraft aus der Differenz (ΔEMG) und aus den bei wenigstens zwei unterschiedlichen Muskelbeanspruchungen ermittelten funktionalen Zusammenhänge zwischen den jeweils wenigstens zwei elektromyographischen Kennwerten ermittelt wird.

21. Verfahren nach einem der vorhergehenden Ansprüche, **dadurch gekennzeichnet, dass** eine muskuläre Beanspruchung ermittelt wird, indem aus der Differenz (ΔEMG) ein funktionaler Zusammenhang zwischen den wenigstens zwei ermittelten elektromyographischen Kennwerten in einem Zustand ohne muskuläre Ermüdung ermittelt wird, aus welchem im statischen Fall eine relative Muskelbeanspruchung bzw. dann, wenn aus einem vorangegangenen Kalibrierschritt die Kraft in einem Zustand ohne muskuläre Ermüdung ermittelt wurde, eine absolute Muskelbeanspruchung ermittelt wird.

22. Verfahren nach einem der Ansprüche 1 bis 20, **dadurch gekennzeichnet, dass** eine relative muskuläre Beanspruchung aus den zu Beginn der zweiten, zu einer definierten muskulären Ermüdung führenden Kalibrierung ermittelten elektromyographischen Kennwerten ermittelt wird, indem fehlende Beanspruchungswerte interpoliert und gegebenenfalls extrapoliert werden oder die Kennwerte mit einer geeigneten Funktion approximiert werden.

## Claims

1. A method to determine a muscular fatigue based on electromyographic measurement values where raw electromyographic signals are recorded, at least two electromyographic parameters are determined from the raw electromyographic signals and/or values derived from them, and where at least two of the electromyographic parameters act in the same direction with a varying force and in a different direction with varying fatigue or in a different direction with a varying force and in the same direction with varying fatigue, in at least one first calibration a functional relationship between the at least two electromyographic parameters is determined, where essentially no muscular fatigue occurs with this calibration, and
in a measurement and analysis phase a difference (ΔEMG) between at least one currently determined electromyographic parameter and the functional relationship between the at least two electromyographic parameters is determined and
a functional relationship between a known muscular fatigue or an actual muscular fatigue which was determined by calibration and the difference (ΔEMG) is determined, and from which a value for muscular fatigue is determined.

2. A method according to claim no. 1, **characterized in that** the raw electromyographic signals are transformed into a frequency spectrum and from the frequency spectrum at least one electromyographic parameter is determined.

3. A method according to any one of the claims 1 or 2, **characterized in that** during the at least one first calibration a variation of force is carried out on the muscle and during the calibration at least the two electromyographic parameters and their functional relationship are determined, where during the calibration essentially no muscular fatigue occurs.

4. A method according to claim no. 3, **characterized in that** with the variation of the force on the muscle, the muscles under examination are loaded simultaneously or one after another with a steadily increasing force and the at least two electromyographic parameters are determined continuously or at least for several different force values.

5. A method according to any one of the preceding claims, **characterized in that** during the first calibration the maximum force level to be developed by the muscle will be set such that it corresponds at least to the highest force which occurs in a subsequent measurement.

6. A method according to any one of the preceding claims, **characterized in that** during the first calibration the force developed by the muscle in relation to the maximum of its voluntary contraction force is determined continuously or at least for several different force values.

7. A method according to any one of the preceding claims, **characterized in that** in case the at least two electromyographic parameters are not linked one-to-one to the muscular force at least one further electromyographic parameter will be determined and used for a one-to-one assignment between parameter and force.

8. A method according to any one of the preceding claims, **characterized in that** from the raw electromyographic signals at least one electrical activity and from the frequency spectrum at least one median frequency or one amplitude relationship between two or more frequency bands is determined.

9. A method according to any one of the preceding claims, **characterized in that** in a second calibration a functional relationship between the at least two electromyographic parameters is determined where the muscle under examination is fatigued in a defined way, continuously or in discrete time intervals a difference (ΔEMG) between a currently determined electromyographic parameter and the functional relationship is established, an actual muscular fatigue is determined from the amount and kind of the relative muscular force during the calibration load and the duration of the calibration load and a functional relationship between the actual muscular fatigue and the difference (ΔEMG) is determined.

10. A method according to claim no. 9, **characterized in that** during the second calibration all muscles under examination are fatigued simultaneously or one after another by a constant force for a defined time, and at least the two electromyographic parameters and their functional relationship are determined in a state of a defined muscular fatigue during the calibration.

11. A method according to the claim no. 9 or 10, **characterized in that** the at least two electromyographic parameters are determined continuously or in defined time intervals.

12. A method according to any one of the claims 9 to 11, **characterized in that** the amount of force to be developed by the muscle is selected such that it provokes a muscular fatigue within a calibration time without forcing the examined muscle close to its performance limits.

13. A method according to claim no. 12, **characterized in that** the relative muscular force developed by the muscle lies between some 20 percent and some 40 percent of the maximum voluntary contraction force of this muscle.

14. A method according to any one of the claims 9 to 13, **characterized in that** at least one additional electromyographic parameter is determined and involved in the functional relationship between the actual muscular fatigue and the difference (ΔEMG).

15. A method according to claim no. 14, **characterized in that** the additional electromyographic parameter is an electromyographic parameter that was already used in the determination of the difference (ΔEMG).

16. A method according to claim no. 14, **characterized in that** the additional electromyographic parameter is an electromyographic parameter which has not been used in determining the difference (ΔEMG) which as much as possible is dependent on the variation of the developed muscular force and as little as possible on the muscular fatigue.

17. A method according to any one of the claims 9 to 16, **characterized in that** for the determination of the actual muscular fatigue an initial muscle load is carried out with a calibration and afterwards at least one second muscle load with a calibration, wherein during the at least one second muscle load the force to be developed by the muscle is smaller or bigger than during the first muscle load and the relationship of the forces developed during the at least two muscle loads to each other is known and that a functional relationship for an actual muscular fatigue in dependence on the difference (ΔEMG) is selected such that it is suitable just as much for the difference between the currently determined electromyographic parameter and the functional relationship between the at least two electromyographic parameters during the first muscle load as well as for the difference between the currently determined electromyographic parameter and the functional relationship between the at least two electromyographic parameters during the at least one second muscle load.

18. A method according to claim no. 17, **characterized in that** the at least one second muscle load is carried out after a complete recovery of the loaded muscle or the muscular fatigue of the muscle under examination is known before the start of the second muscle load.

19. A method according to claim no. 17 or 18, **characterized in that** the relative muscular force during the second muscle load is doubled in comparison with the first muscle load.

20. A method according to any one of the claims 17 to 19, **characterized in that** a relative muscular force is determined from the difference (ΔEMG) and from the functional relationships determined between the at least two electromyographic parameters each from at least two different muscle loads.

21. A method according to any one of the preceding claims, **characterized in that** a muscle load is determined by determining a functional relationship between the at least two determined electromyographic parameters in a condition without muscular fatigue from the difference (ΔEMG), and from which, in a static case, a relative muscle load is determined or, respectively, an absolute muscle load, if the force was determined from a preceding calibration step in a condition without muscular fatigue.

22. A method according to any one of the claims 1 to 20, **characterized in that** a relative muscle load is determined by interpolation, and, if necessary, extrapolation of missing load values or by approximation of the parameters with the help of a suitable function from the electromyographic parameters determined at the beginning of the second calibration leading to a defined muscular fatigue.

## Revendications

1. Procédé pour déterminer une fatigue musculaire à partir de valeurs de mesures tirées d'un électromyogramme, tandis que des signaux bruts de l'électromyogramme sont enregistrés,
Au moins deux paramètres caractéristiques de l'électromyogramme sont simultanément déterminés, à partir des signaux bruts de l'électromyogramme et / ou des valeurs qui en sont déduites, tandis qu'au moins deux des paramètres de l'électromyogramme se comportent dans le même sens en cas de variation de la fatigue, ou dans un sens opposé en cas de variation de la force, et dans le même sens en cas de variation de fatigue,
Dans au moins un de ces étalonnages, un rapport fonctionnel entre au moins deux paramètres caractéristiques de l'électromyogramme est déterminé, alors qu'aucune fatigue musculaire importante n'apparaît lors de cet étalonnage, et lors d'une phase de mesure et d'exploitation, une différence (ΔEMG) est déterminée qui permet de calculer une valeur de fatigue musculaire.

2. Procédé selon l'exigence 1, **caractérisé par le fait que** les signaux bruts de l'électromyogramme sont transformés dans un spectre de fréquence, et qu'au moins un paramètre caractéristique est déterminé à partir de cette fréquence de spectre.

3. Procédé selon l'exigence 1, **caractérisé par le fait qu'**au moins lors d'un des premiers étalonnages une variation de force a été effectuée, et que lors de l'étalonnage, les deux paramètres caractéristiques de l'électromyogramme au moins et leur rapport fonctionnel sont établis, tandis que lors de l'étalonnage aucune fatigue musculaire n'apparaît.

4. Procédé selon l'exigence 3, **caractérisé par le fait que** lors d'une variation de force au niveau du muscle, les muscles à analyser en même temps ou successivement sont sollicités avec une force en croissance permanente et que les deux paramètres caractéristiques de l'électromyogramme au moins sont déterminés en continu ou suite plusieurs valeurs différentes de forces.

5. Procédé selon une des exigences précédentes, **caractérisé par le fait que** lors du premier étalonnage, une force fournie par le muscle est définie de manière à ce qu'elle corresponde au moins à la force la plus élevée lors d'une mesure annexe.

6. Procédé selon une des exigences précédentes, **caractérisé par le fait que**, lors du premier étalonnage, la force fournie par le muscle, en fonction de sa force de contraction volontaire est déterminée en continu ou au moins suite à différentes valeurs de forces.

7. Procédé selon une des exigences précédentes, **caractérisé par le fait qu'**ensuite, si les deux paramètres caractéristiques au moins ne s'accompagnent pas de manière univoque de force musculaire, au moins un paramètre caractéristique de l'électromyogramme est déterminé et est utilisé pour classer et faire la distinction de manière univoque entre un paramètre caractéristique et une force.

8. Procédé selon une des exigences précédentes, **caractérisé par le fait qu'**au moins une activité électrique est produite à partir des signaux bruts de l'électromyogramme, qu'au moins une fréquence médiane est produite à partir du spectre de fréquence, ou qu'un rapport d'amplitude est produit à partir de deux ou plusieurs bandes de fréquence.

9. Procédé selon une des exigences précédentes, **caractérisé par le fait que**, dans un deuxième étalonnage, un rapport fonctionnel entre au moins deux paramètres caractéristiques de l'électromyogramme est déterminé, tandis que le muscle à examiner est défini comme fatigué, de manière consécutive ou dans des intervalles temporels légers, une différence ( ΔEMG) entre un paramètre caractéristique actuellement défini et le rapport fonctionnel est déterminée, une fatigue musculaire réelle est établie à partir de la hauteur et du mode de force musculaire relative lors de la sollicitation pour l'étalonnage, et un rapport fonctionnel entre la fatigue musculaire réelle et la différence (ΔEMG) est déterminé.

**10.** Procédé selon l'exigence 9, **caractérisé par le fait que** lors du deuxième étalonnage, tous les muscles à analyser ont été fatigués simultanément ou successivement avec une force constante pendant un temps défini, et pendant l'étalonnage au moins deux paramètres caractéristiques de l'électromyogramme ainsi que leur rapport fonctionnel dans un état donné de fatigue musculaire, ont été déterminés.

**11.** Procédé selon l'exigence 9 ou 10, **caractérisé par le fait que** les deux paramètres caractéristiques de l'électromyogramme au moins sont définis en continu ou à des intervalles de temps définis.

**12.** Procédé selon les exigences 9 à 11, **caractérisées par le fait que** la force apportée par le muscle est choisie à un niveau qui provoque, à l'intérieur d'un temps d'étalonnage, une fatigue musculaire, sans entraîner les muscles analysés à proximité des frontières de sa performance.

**13.** Procédé selon l'exigence 12, **caractérisé par le fait que** la force musculaire relative fournie par le muscle se trouve entre environ 20% et 40% de la force de contraction volontaire maximale de ce muscle.

**14.** Procédé selon les exigences 9 à 13, **caractérisés par le fait qu'**au moins un autre paramètre caractéristique de l'électromyogramme est déterminé et est intégré dans le rapport fonctionnel entre la fatigue musculaire réelle et la différence (ΔEMG).

**15.** Procédé selon l'exigence 14, **caractérisé par le fait que** le paramètre caractéristique de l'électromyogramme suivant est un paramètre caractéristique de l'électromyogramme déjà utilisé pour déterminer la différence (ΔEMG).

**16.** Procédé selon l'exigence 14, **caractérisé par le fait que** le paramètre caractéristique de l'électromyogramme suivant est un paramètre caractéristique de l'électromyogramme qui n'a pas été utilisé pour déterminer la différence (ΔEMG), qui peut être fortement dépendant de la variation de la force musculaire utilisée et faiblement dépendant de la fatigue musculaire.

**17.** Procédé selon une des exigences 9 à 16, **caractérisé par le fait que** pour la détermination de la fatigue musculaire réelle, une première sollicitation musculaire avec un étalonnage, suivie d'au moins une deuxième sollicitation musculaire avec étalonnage est effectuée, au cours de laquelle cependant, lors de la deuxième sollicitation musculaire, la force fournie par le muscle est plus faible ou plus élevée que lors de la première sollicitation musculaire, et que la relation des forces fournies lors des deux sollicitations est connue, et qu'un rapport fonctionnel pour une fatigue musculaire effective en relation avec la différence (ΔEMG) qui est choisie, et qu'il est tout aussi approprié à la différence entre le paramètre caractéristique de l'électromyogramme actuellement déterminé et le rapport fonctionnel entre au moins deux paramètres caractéristiques de l'électromyogramme lors de la première sollicitation musculaire, qu'à la différence entre le paramètre caractéristique actuellement déterminé et le rapport fonctionnel entre au moins deux paramètres caractéristiques de l'électromyogramme lors d'au moins une des deux sollicitations musculaires.

**18.** Procédé selon l'exigence 17, **caractérisé par le fait qu'**au moins une deuxième sollicitation musculaire est réalisée après un repos total du muscle sollicité, ou que la fatigue musculaire du muscle à examiner est connue avant le commencement de la deuxième sollicitation musculaire.

**19.** Procédé selon l'exigence 17 ou 18, **caractérisé par le fait que** la force musculaire relative lors de la deuxième sollicitation musculaire est le double de la première sollicitation musculaire.

**20.** Procédé selon une des exigences 17 à 19, **caractérisé par le fait qu'**une force musculaire relative issue de la différence (ΔEMG) et d'au moins deux sollicitations musculaires des rapports fonctionnels déterminés entre chacun des paramètres caractéristiques de l'électromyogramme est déterminée.

**21.** Procédé selon une des exigences précédente, **caractérisé par le fait qu'**une sollicitation musculaire est déterminée, tandis qu'un rapport fonctionnel entre au moins deux paramètres caractéristiques déterminés issu de la différence (ΔEMG), dans un état sans fatigue musculaire, est déterminé, à partir duquel une sollicitation musculaire relative dans un cas statique ou ensuite, si la force a été déterminée dans un état sans fatigue musculaire à partir d'une étape d'étalonnage antérieure, si une sollicitation musculaire absolue est déterminée.

22. Procédé selon une des exigences 1 à 20, **caractérisé par le fait qu'**une sollicitation musculaire relative issue des paramètres caractéristiques de l'électromyogramme définis établis au début du deuxième étalonnage avec une fatigue musculaire donnée, est déterminée, tandis que les valeurs de sollicitation manquantes sont interpolées et extrapolées le cas échéant, ou que les paramètres caractéristiques sont définis approximativement à l'aide d'une fonction appropriée.

Figur 1

relative statische Muskelkraft [1]

Figur 2

Figur 3

Figur 4

MF = f(EA) - nicht erm. Musk.
MF = f(EA) - 20% musk. Ermüdung
MF = f(EA) - 40% musk. Ermüdung
MF = f(EA) - 60% musk. Ermüdung

Figur 5

□□□ Messwerte Fr = 0,25
○○○ Messwerte Fr = 0,45
- - - Approx. Fr = 0,25
——— Approx. Fr = 0,45

Figur 6

Figur 7

Figur 8

EP 2 067 439 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **Hägg ; Luttmann ; Jäger.** Methodologies for evaluating electromyographic field data in ergomomics. *Journal of Electromyography and Kinesiology,* 2000, vol. 10, 301-312